# EUROPÄISCHE PATENTSCHRIFT

(11) **EP 3 083 549 B1**
(45) Veröffentlichungstag und Bekanntmachung des Hinweises auf die Patenterteilung: **20.09.2017**
(21) Anmeldenummer: 14809815.5
(22) Anmeldetag: 05.12.2014
(51) Int. Cl.: C07C 67/39

(54) **VERFAHREN ZUR HERSTELLUNG VON UNGESÄTTIGTEN ESTERN AUSGEHEND VON ALDEHYDEN DURCH DIREKTE OXIDATIVE VERESTERUNG**
METHOD FOR PRODUCING UNSATURATED ESTERS FROM ALDEHYDES BY DIRECT OXIDATIVE ESTERIFICATION
PROCÉDÉ DE FABRICATION D'ESTERS NON SATURÉS À PARTIR D'ALDÉHYDES PAR ESTÉRISATION OXYDATIVE DIRECTE

(30) Priorität: 20.12.2013 EP 13198863
(43) Veröffentlichungstag der Anmeldung: 26.10.2016
(73) Patentinhaber: Evonik Röhm GmbH, 64293 Darmstadt (DE)
(72) Erfinder: KRILL, Steffen, 64367 Mühltal (DE); LYGIN, Alexander, 64347 Griesheim (DE); BALDUF, Torsten, 64319 Pfungstadt (DE); BURGHARDT, Rudolf, 64287 Darmstadt (DE); TEPPERIS, Andreas, 64732 Bad König (DE); GRÖMPING, Matthias, 64295 Darmstadt (DE)
(86) Internationale Anmeldenummer: PCT/EP2014/076682
(87) Internationale Veröffentlichungsnummer: WO 2015/091018

(56) Entgegenhaltungen:
- EP-A1- 2 210 664

## Beschreibung

### Gebiet der Erfindung

Die vorliegende Erfindung betrifft ein Verfahren zur Herstellung von Methylmethacrylat durch direkte oxidative Veresterung von Methacrolein.

Methylmethacrylat wird in großen Mengen zur Herstellung von Polymeren und Copolymeren mit anderen polymerisierbaren Verbindungen eingesetzt. Weiterhin ist Methylmethacrylat ein wichtiger Baustein für diverse, auf Methacrylsäure (MAS) basierenden Spezialester, die durch Umesterung mit dem entsprechenden Alkohol hergestellt werden können.

Hieraus ergibt sich ein großes Interesse an möglichst einfachen, wirtschaftlichen und umweltschonenden Herstellungsverfahren für diesen Ausgangsstoff.

### Stand der Technik

Methylmethacrylat (MMA) wird heute überwiegend ausgehend von Blausäure und Aceton über das entstehende Acetoncyanhydrin (ACH) als Zentralintermediat hergestellt. Dieses Verfahren hat den Nachteil, dass sehr große Mengen an Ammoniumsulfat erhalten werden, deren Aufbereitung mit sehr hohen Kosten verbunden ist. Weitere Verfahren, die eine andere Rohstoffbasis als ACH verwenden, sind in der einschlägigen Patentliteratur beschrieben und mittlerweile im Produktionsmaßstab realisiert worden. In diesem Zusammenhang werden heute auch C-4 basierte Rohstoffe wie Isobutylen oder tert-Butanol als Edukte verwendet, die über mehrere Verfahrensstufen in die gewünschten Methacrylsäurederivate umgewandelt werden.

Hierbei wird im Allgemeinen Isobutylen oder tert-Butanol zu Methacrolein in einer ersten Stufe oxidiert, welches anschließend zu Methacrylsäure mit Sauerstoff umgesetzt wird. Die erhaltene Methacrylsäure wird nachfolgend mit Methanol in MMA überführt. Nähere Einzelheiten zu diesem Verfahren sind unter anderem in Ullmann's Encyclopedia of Industrial Chemistry 2012, Wiley-VCH Verlag GmbH & Co. KGaA, Weinheim, Methacrylic Acid and Derivatives, DOI: 10.1002/14356007.a16_441.pub2 dargelegt.

In einem weiteren Verfahren wird MMA durch Oxidation von Isobutylen oder tert-Butanol mit Luftsauerstoff in der Gasphase am heterogenen Kontakt zu Methacrolein und anschließender oxidativer Veresterungsreaktion von Methacrolein unter Verwendung von Methanol erhalten. Dieses von ASAHI entwickelte Verfahren ist unter anderem in den Druckschriften US 5,969,178 und US 7,012,039 beschrieben. Nachteil dieses Verfahrens ist insbesondere ein sehr hoher Energiebedarf.

In US 5,969,178 ist ein Verfahren zur oxidativen Umsetzung von Isobuten oder tert-Butanol zu Methacrolein und die folgende oxidative Veresterung zu MMA beschrieben. In dieser zweiten Stufe wird eine flüssige, im Wassergehalt reduzierte Mischung aus Methacrolein und Methanol mit molekularem Sauerstoff und einem Palladiumkatalysator umgesetzt, wobei dieser zumeist auf einem Träger als Palladium- Blei-Katalysator vorliegt. Gemäß US 6,040,472 führt ein solcher Pd-Pb-Katalysator bei einem optimalen Palladium-Anteil von 5 % zu einer MMA-Selektivität von bis 91 % und zu einer Raum-Zeit-Ausbeute von 5,3 mol MMA / h*kg Katalysator. Palladium(-Blei) Katalysatoren haben jedoch den Nachteil, dass es in einem kontinuierlichen Betrieb zu hohen Verlusten der Bleikomponente kommt (so genanntes Leaching). Auf der einen Seite, führt dies zu einer aufwendigen Abwasserentsorgung, auf der anderen Seite müssen Bleisalze dem System kontinuierlich zugeführt werden.

EP 2 177 267 und EP 2 210 664 beschreiben Nickeloxid-Katalysatoren mit einem Goldanteil zwischen 1 und 80 mol%, die auf einem Trägermaterial vorgelegt werden, für die oxidative Veresterung von Aldehyden zu Estern. Diese Katalysatoren werden mit einem Durchmesser zwischen 10 und 200 µm eingesetzt. Insbesondere liegen diese Partikel mit einer Schalenstruktur vor, bei der das Nickeloxid sich auf der Oberfläche und das Gold auf einer innenliegenden Schicht befindet. Diese Katalysatoren führen bestenfalls zu einer MMA-Selektivität von bis 97,1 % bei einer Raum-Zeit-Ausbeute von 9,6 mol MMA / h*kg Katalysator.

EP 2 210 664 offenbart dazu eine spezielle Variante, bei der Katalysatorpartikel im Nanometerbereich auf einen Supportpartikel mit einem Durchmesser zwischen 10 und 200 µm aufgebracht sind. In einer Variante hat dieser Supportpartikel eine Größe von 3 mm. Der Katalysator kann auch zylinderförmig oder in Wabenform in einem Festbettreaktor vorgelegt werden. Weiter wird die Prozessführung in einer solchen Reaktorvariante nicht beschrieben.

In EP 1 393 800 werden Goldpartikel oder goldhaltige Partikel mit einem Durchmesser von kleiner 6 nm auf einem Trägermaterial, insbesondere auf einem Metalloxid, als Katalysator beschrieben. Selektivitäten zu MMA von bis zu 93 % und Raumzeitausbeuten von bis zu 50,7 mol MMA / h*kg Katalysator bei einem Goldgehalt der Katalysatorpartikel von 4,5 mol% erhalten. Darüber hinaus ist der Offenbarungsgehalt dem der EP 2 210 664 analog.

Weiterhin wird der auf einem Träger (z.B. SiO₂) aufgebrachte Katalysator in Form eines Suspensionskatalysators eingesetzt. Bei der Suspensionskatalyse wird in einem entsprechenden Reaktortyp, z.B. einem Slurry-Type Reaktor, ein pulverförmiger Katalystor als Feststoff gerührt und mit den Edukten (in diesem Fall dem Aldehyd, dem Alkohol und einem Sauerstoff-haltigen Gas) in Kontakt gebracht, wobei das Gemisch mechanisch umgewälzt wird und der Katalysator mehr oder weniger großem mechanischen Stress ausgesetzt wird. Damit ist zwar die Durchmischung mit den Reaktanten sehr gut, es führt jedoch auch zu einem Austrag von Katalysatormengen bzw. Katalysatorabrieb und damit zu einem schnelleren Verbrauch des Katalysators. So müssen bei einem ähnlichen Verfahren in flüssiger Phase gemäß JP 06080611A mittels einer Querschnittsfiltration Katalysatoren zurück gewonnen werden. Dabei muss aufgrund des Abriebs ein ganzes Kornsprektrum abgetrennt werden und nicht nur das Katalystorkorn in seiner ursprünglich eingesetzten Form und Größe. Bei der Filtration des Katalysatorabriebs kommt es schnell zur Verstopfung des Filters und es müssen aufwändige Steuerungen zur Rückspülung und Regenerierung der Filter installiert werden. Anderseits ergibt sich das Problem der Ausschleusung des Feinanteils oder der Anreicherung im Reaktionskessel.

Gemäß CN 1931824A kann dieses Problem einer zusätzlichen Filtration durch Vorlage der Palladium-Blei-Katalysatoren auf einem Trägermaterial mit einem Durchmesser zwischen 2 und 5 mm in einem Festbett gelöst werden. Grundsätzlich führt dieses Vorgehen jedoch zu einer verminderten Raum-Zeit-Ausbeute und anderen Nachteilen.

Als Nebenprodukt der MAL-Synthese bildet sich Methacrylsäure und der pH-Wert des Reaktiongemisches sinkt entsprechend. Dies führt zu weiteren Problemen. So wird mit sinkendem pH-Wert in steigenden Mengen das Nebenprodukt 1,1-Dimethoxyisobuten (DMI) als Acetal aus Methacrolein und Methanol gebildet. Damit steht ein Teil des Methacroleins in Form eines Dimethylacetals für die weitere Umsetzung zum MMA nicht mehr zur Verfügung, und die Raumzeitausbeute der MMA-Synthese sinkt entsprechend. Das Dimethylacetal bereitet darüber hinaus in der darauf folgenden destillativen Aufarbeitung des MMA Probleme. Außerdem wirkt ein Gemisch mit einem zu geringen pH-Wert negativ auf die Stabilität und Lebensdauer des eingesetzten Katalysators (Leaching, Änderung der Porenstruktur des Katalysators usw.). So lehrt in Bezug auf die Untergrenze von pH = 5 die JP 2003048863, dass man eine basische Lösung zum Ausgleich des pH-Werts zugeben kann. Diese basische Lösung, z.B. in Form einer NaOH-Lösung, selbst weist in der Regel einen pH-Wert größer 10 auf.

Bei gemäß dem Stand der Technik vor allem für die oxidative Veresterung eingesetzten Slurry-Type-Reaktoren handelt es sich um einen oder mehrere in Kaskade geschaltete Reaktoren, die mit einem Rührer umgewälzt werden. In den Reaktoren kann wie ausgeführt durch Kontrolle des pH-Werts die Zugabe der Base, mit dem Ziel einen konstanten pH einzuhalten, gesteuert werden. Der Kesselreaktor wird mittels eines Wärmetauschers abgekühlt, um die Exotherme der Reaktion abzuführen. Eine homogene Wärmeverteilung im Reaktor und an der Oberfläche des Katalysators - insbesondere beim nicht-Vorliegen von so genannten "hot spots" - ist sehr wichtig für das Erzielen von hohen Selektivitäten und für eine optimale Nutzung des Katalysators. Gasförmiger Sauerstoff muss als Reagenz dem System kontinuierlich zugeführt werden. Aufgrund der Explosionsgefahr muss Sauerstoff zusätzlich mit einem inerten Gas, wie z.B. Stickstoff, verdünnt werden. Daraus resultiert wiederum ein großer Gasstrom, der gleichzeitig mit der Flüssigkeit in Kontakt mit der Katalysatoroberfläche gebracht werden muss. Geringere Wärmeleitfähigkeit von Gasen im Vergleich zu Flüssigkeiten führt zu einer schlechteren Wärmeverteilung.

Zusammengefasst sind die folgenden Aspekte der Verfahren gemäß Stand der Technik verbesserungswürdig:
- mechanischer Abrieb des Katalysators
- Die kontinuierliche Trennung des Reaktionsgemisches vom Katalysator
- Leaching, und die daraus resultierende relativ geringe Lebensdauer des Katalysators
- Die Wärmeabfuhr, sowie die homogene Wärmeverteilung im Reaktor, bzw. an Katalysatoroberfläche
- Resultierend u.a. aus den benannten Nachteilen die Ausbeute und die Selektivität des Prozesses

### Aufgabe

In Anbetracht des Standes der Technik ist es daher Aufgabe der vorliegenden Erfindung, ein technisch verbessertes Verfahren zur oxidativen Veresterung von Methacrolein zur Verfügung zu stellen, das nicht mit den Nachteilen herkömmlicher Verfahren behaftet ist.

Insbesondere sollen Verfahren des Standes der Technik dergestalt verbessert werden, dass weniger 1,1-Dimethoxyisobuten, weniger Michael-Addukt und weniger Methacrylsäure in freier Form während der oxidativen Veresterung von Methacrolein gebildet werden. 1,1-Dimethoxyisobuten bildet sich dabei vor allem in einem zu saurem Medium, während das Michael-Addukt in einem eher zu basischen Medium als Nebenprodukt entsteht.

Weiterhin soll das verbesserte Verfahren über eine lange Standzeit, bei gleichzeitig nahezu konstanten und hohen Selektivitäten und Raum-Zeit-Ausbeuten durchführbar sein.

Weiterhin soll das Verfahren gegenüber dem Stand der Technik kostengünstig, insbesondere ohne größere Katalysatorverluste durch Abrieb oder Austrag durchführbar sein.

Ferner sollte das Verfahren mit relativ einfachen und kostengünstigen Anlagen durchgeführt werden können. Die Anlagen sollten demgemäß mit geringen Investitionskosten verbunden sein. Hierbei sollen die Anlagen einfach zu warten sein, geringen Unterhaltskosten verursachen und sicher zu betreiben sein.

Weitere nicht explizit genannte Aufgaben ergeben sich aus dem Gesamtzusammenhang der nachfolgenden Beschreibung und der Ansprüche.

### Lösung

Gelöst werden die Aufgaben durch ein neuartiges Verfahren zur Herstellung von Methylmethacrylat aus Methacrolein in einer kontinuierlichen oxidativen Veresterungsreaktion mit Sauerstoff und Methanol. Erfindungsgemäß wird dieses Verfahren in flüssiger Phase bei einem Druck von 2 bis 100 bar mit einem heterogenen Katalysator durchgeführt. Bei dem heterogenen Katalysator handelt es sich um geträgerte goldhaltige Nanopartikel mit einer Teilchengröße kleiner 20 nm, bevorzugt zwischen 0,2 und 20 nm. Insbesondere ist das erfindungsgemäße Verfahren dadurch charakterisiert, dass die flüssige Phase dem Reaktor kontinuierlich entnommen wird und der pH-Wert nach der Entnahme mittels Zugabe einer basischen Lösung auf einen pH-Wert zwischen 5 und 9, bevorzugt zwischen 6 und 8,5 und besonders bevorzugt zwischen 6,5 und 8,0 eingestellt wird. Diese entnommene flüssige Phase mit einem pH-Wert zwischen 5 und 9 wird anschließend zu mindestens 50 %, bevorzugt zu mindestens 70 % und besonders bevorzugt zu mindestens 90 % zurück in den Reaktor geführt.

Eine solche erfindungsgemäße Regelung des pH-Werts des Reaktionsgemisches außerhalb des Reaktors und das zumindest teilweise Zurückführen des Gemischs in den Reaktor sind gegenüber dem Stand der Technik eine überraschend einfache Lösung zur Verbesserung der Ausbeute bzw. der Selektivität der Reaktion.

Die Entnahme der flüssigen Phase kann kontinuierlich, semikontinuierlich oder batchweise, bevorzugt kontinuierlich erfolgen.

Bei pH-Werten oberhalb von 9 tritt vor allem eine Michael-Addition des Methanols an die Doppelbindung als Nebenreaktion auf. Auch diese wirkt sich negativ auf die Raumzeitausbeute und die Selektivität zu MMA aus. Außerdem wirkt sich ein Medium mit einem pH-Wert größer 9 negativ auf die Stabilität und Lebensdauer des eingesetzten Katalysators (Leaching, Änderung der Porenstruktur des Katalysators usw.) aus. Dies kommt insbesondere zum Tragen, wenn eine solche basische Lösung direkt mit dem Katalysator in Kontakt kommt. Diese Probleme konnten durch die erfindungsgemäße Regulierung des pH-Werts überraschend effizient vermieden bzw. auf ein Minimum reduziert werden.

In einer bevorzugten Ausführungsform handelt es sich bei dem Reaktor um einen Festbettreaktor, in dem sich die goldhaltigen Nanopartikel auf Trägerpartikeln mit einem Gesamtdurchmesser zwischen 0,2 und 20 mm, die wiederum im Festbett vorgelegt sind, befinden. Bei einem Festbettreaktor ist der Katalysator immobilisiert und es erfolgt in der Regel ein Durchströmen des Reaktors mit der Reaktionslösung von unten nach oben oder umgekehrt. Bevorzugt wird für einen Festbettreaktor kein Rührer benötigt.

Eine Einstellung des pH-Werts durch Zugabe einer basischen Lösung direkt in den Reaktor, wie es gemäß Stand der Technik für Rührreaktoren beschrieben ist, ist bei einem Festbettreaktor aufgrund der geringeren Mischung der basischen Lösung mit der Reaktionslösung negativ für die Katalysatorstandzeit und die Selektivität der Reaktion. Es kommt zu lokalen Unterschieden des pH-Werts, insbesondere in der Nähe der Einleitungsstelle der basischen Lösung.

Überraschend wurde gefunden, dass es mittels des erfinderischen Verfahrens auch möglich ist, den pH-Wert der Reaktionslösung für die Reaktion in einem Festbettreaktor einzustellen und dabei gleichzeitig lange Katalysatorstandzeiten, eine hohe Selektivität und sehr gute Ausbeuten zu erzielen.

Überraschend wurde außerdem gefunden, dass durch das erfindungsgemäße Verfahren nicht nur die Selektivität durch Unterdrücken von Nebenreaktionen gesteigert werden kann, sondern dass zusätzlich die Raum-Zeit-Ausbeute gegenüber Verfahren des Standes der Technik auch in Reaktoren mit immobilisierten Katalysatoren erhöht werden konnte. Letztere geht unter anderem auf einen weiteren überraschenden Effekt des erfindungsgemäßen Verfahrens zurück. Durch das Fahren eines großen Teils der flüssigen Phase im Kreislauf wird das gesamte Angebot an gelöstem Sauerstoff an der Katalysatoroberfläche pro Zeiteinheit größer. Dies wäre anders, wenn die flüssige Phase kontinuierlich dem Reaktor entzogen und nicht zurückgeführt würde. Bei einem solchen Verfahren müssten zur Realisierung der gleichen gelösten Sauerstoffmengen in der flüssigen Phase relevant höhere Sauerstoffpartialdrücke (z.B. in Form höherer Sauerstoffanteil im Feed-Gasgemisch) in den Reaktor gedrückt werden. Dies geht jedoch aufgrund der potentiellen Explosivität der Reaktionsmischung und der größeren Gasphase in einem solchen Fall mit hohen Sicherheitsrisiken einher.

Sollte man andererseits in einem Verfahren ohne Kreislaufführung auf eine Erhöhung des O₂-Partialdrucks verzichten, dann ist das Sauerstoffangebot pro Zeiteinheit direkt am Katalysator vermindert und die Raum-Zeit-Ausbeute dementsprechend geringer. Die Löslichkeit von Sauerstoff ist insgesamt sehr gering. So ist der Molbruch für 1 Atmosphäre O₂-Druck bei 50 °C in Wasser 0,17*10⁻⁴ und in Methanol 4,01*10⁻⁴.

In einer alternativen, gleichsam bevorzugten Ausführungsform befinden sich die goldhaltigen Nanopartikel auf Trägerpartikeln mit einem Gesamtdurchmesser kleiner 0,2 mm. In dieser Ausführungsform wird die Reaktion in einem Rührreaktor, also einem Reaktor mit Rührvorrichtung durchgeführt. Die Katalysatorpartikel werden dabei in der Reaktionslösung entsprechend umgewälzt.

In einer weiteren, gleichsam bevorzugten Ausführungsform befinden sich die goldhaltigen Nanopartikel ebenfalls auf Trägerpartikeln mit einem Gesamtdurchmesser kleiner 0,2 mm. In dieser Ausführungsform wird jedoch die Reaktion in einer Wirbelschicht-artigen Vorrichtung durchgeführt. Die Katalysatorpartikel werden dabei in einer Katalysatorschüttung mit einem durchströmenden flüssigen Strom entsprechend umgewälzt und verteilt.

Unabhängig von der Ausführungsform weist das erfindungsgemäße Verfahren gegenüber dem Stand der Technik gleich mehrere Vorteile auf:
- Der Abrieb an Katalysatoren wird bei konstant gehaltenem pH-Wert und durch die Wahl des verwendeten geträgerten Gold-haltigen Katalysator minimiert, bzw. in der Ausführungsform eines Festbettreaktors nahezu ganz vermieden. Dies verlängert zum einen die Katalysatorstandzeit und vermeidet zum anderen potentielle Probleme bei der Filtration, wie z.B. das Zusetzen der Filter.
- Im Falle der Ausführungsform der Erfindung in Form eines Festbettreaktors ist die Temperaturkontrolle der exothermen Reaktion durch einen effizienten Wärmeaustausch im erfindungsgemäßen Verfahren besonders gut gewährleistet.
- Durch die Einstellung des pH Wertes mit einer basischen Lösung (pH > 9) außerhalb des Reaktors wird der direkte Kontakt des Katalysators mit einem zu basischen, d.h. mit einem Medium mit einem pH-Wert größer 9, oder zu aciden Medium, d.h. mit einem pH-Wert kleiner 5, wie es bei der Bildung größerer Mengen Methacrylsäure entstehen kann, vermieden. Daraus resultieren eine höhere Katalysatorstabilität und damit längere Lebensdauer bzw. Standzeit.

In der Regel wird die oxidative Veresterungsreaktion bei einem Druck im Bereich von 2 bis 50 bar und einer Temperatur im Bereich von 10 bis 200 °C durchgeführt. Es ist dabei insbesondere vorteilhaft, die Reaktion bei einem höheren Druck als im Stand der Technik beschrieben, durchzuführen. Für die Aufarbeitung ist ein erhöhter Reaktionsdruck von Vorteil, da man überraschend weniger Kühlenergie benötigt. Weiterhin ist die Aufarbeitung bei erhöhten Drucken deutlich einfacher und man kann Stoffverluste deutlich reduzieren. Diese Effekte können dadurch erklärt werden, dass es sich bei Methylmethacrylat (MMA), Methacrolein (MAL), Methanol und dem Nebenprodukt Methylformiat um leichtsiedende Komponenten handelt. Bevorzugt werden die gasförmigen und die flüssigen Edukte gleichzeitig, bevorzugt in den oberen Teil des Reaktors im Sinne eines Rieselbettreaktors, zugeführt. So erreicht man eine sehr enge, homogene Verteilung von Gas und Flüssigkeit, womit wiederum eine hohe Selektivität und gleichbleibende Aktivität des Katalysators bewirkt werden.

Die Abtrennung der verbleibenden Gasphase kann dann beispielsweise vor der Rückführung der flüssigen Phase in den Reaktor, z.B. direkt vor dem Behälter zur pH-Wert-Einstellung, mittels des Phasentrenners, erfolgen.

Weiterhin erfolgt die oxidative Veresterungsreaktion bevorzugt mit einem frisch in den Reaktor geleiteten Gemisch aus Methanol und Methacrolein , das einen Anteil des Methacrolein bezogen auf das Gemisch im Bereich zwischen 20 und 60 Gew%, bevorzugt zwischen 25 und 40 Gew% enthält.

Bei den beschriebenen Verfahren, bei denen nur ein mindestens 50 %iger, bevorzugt mindestens 70%iger und besonders bevorzugt mindestens 90 %iger Anteil der flüssigen Phase in den Reaktor zurückgeleitet wird, wird der restlichen Anteil der flüssigen Phase einer Aufarbeitung zur Isolierung des gewonnen MMA zugeführt.

In einer alternativen Ausführungsform der vorliegenden Erfindung wird die flüssige Phase zu 100 % zurück in den Reaktor geführt. In dieser Ausführungsform wird an einer anderen Stelle des Reaktors - und nicht in der Kreislaufführung - flüssige Phase zur weiteren Aufarbeitung kontinuierlich, semikontinuierlich oder batchweise entnommen.

Weiterhin ist es bevorzugt, diese dem System kontinuierlich entnommene flüssige Phase, die hauptsächlich Methacrolein, MMA und Methanol enthält, zu entwässern und das nicht umgesetzte, an Wasser reduzierte Methacrolein zusammen mit Methanol zurück in den Reaktor zu führen. Ein solches Vergehen kann beispielsweise in US 7,012,039 nachgelesen werde.

Das dem Reaktor aus einer Vorstufe oder einem Vorratsbehälter zugeführte Methacrolein kann vor der Zuleitung in den Reaktor, bevorzugt in Gegenwart von Methanol, destillativ entwässert werden. Das so entwässerte MAL und Methanol haltige Gemisch kann dann in den Reaktor geleitet werden. Alternativ kann dieses Methacrolein auch direkt in die beschriebene Destillationsvorrichtung zur Entwässerung der aus dem Reaktor entnommenen flüssigen Phase geleitet werden. Auf diese Weise reicht eine Destillationsvorrichtung zur Entwässerung beider Phasen aus. Diese Aspekte einer solchen Ausführungsform der Erfindung können beispielsweise in der am 26.09.2013 hinterlegten europäischen Patentanmeldung mit dem Aktenzeichen EP 13186137 nachgelesen werden.

Aufgrund der Explosionsgefahr hat es sich als besonders vorteilhaft erwiesen, das Verfahren so durchzuführen, dass die Sauerstoffkonzentration im Abgas aus dem System weniger als 8 Vol% beträgt. Dies kann durch eine entsprechende Regelung des Sauerstoffgehalts in dem zur oxidativen Veresterung zugeleiteten Gasgemischs eingestellt werden. Dazu kann z.B. bei Bedarf Luft mit einem weiteren, in der Reaktion inerten Gas, wie z.B. Stickstoff, Kohlendioxid oder Argon, vor der Zuleitung verdünnt werden. Es können auch Gasgemische aus solchen Gasen und reinem Sauerstoff bereitgestellt werden. Bevorzugt wird der Sauerstoffgehalt des Abgases kontinuierlich mittels einer Sonde bestimmt, und die Gaszusammensetzung und/oder Gasmenge in der Zuluft entsprechend automatisch geregelt.

Weiterhin ist es in der Ausführungsform des erfindungsgemäßen Verfahrens mit einem Festbettreaktor vorteilhaft, diesen Festbettreaktor mit einem Katalysatorschüttungsvolumen/Reaktorvolumen Verhältnis größer 0,01, bevorzugt 0,1 und besonders bevorzugt größer 0,2 zu betreiben.

### Exemplarische Darstellung einer Ausführung der Erfindung:

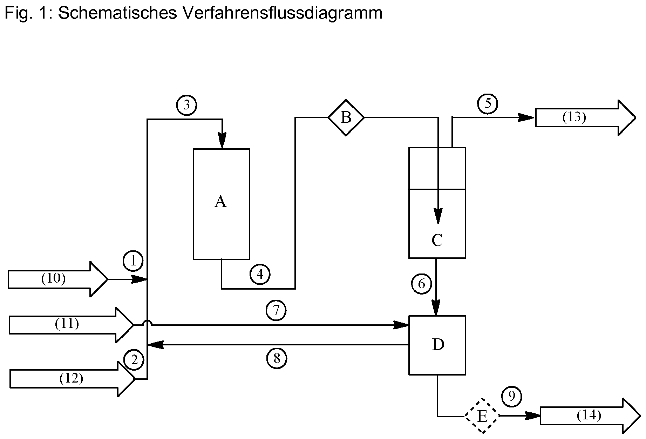

Eine mögliche Ausführungsform der Erfindung mit einem Festbettreaktor ist in Fig. 1 abgebildet. Sauerstoff oder O₂-haltiges Gas wird über Leitung 1 und eine Methacrolein/Methanol Lösung über Leitung 2 dem Reaktor A zugeführt. Die Komponenten werden gemischt und das resultierende heterogene gasförmige/flüssige Gemisch wird weiter über Leitung 3 dem mit Außenmantel temperierbaren Festbettreaktor A zugeführt. Dabei können sowohl das Gas/Flüssigkeit Gemisch als auch die einzelnen Komponente von oben als auch von unten dem Reaktor zugeführt werden. Der Reaktor ist mit dem entsprechenden Festbettkatalysator gefüllt, wobei die Größe der einzelnen Katalysatorpartikeln groß genug sein muss (D > 0,2 mm) um Druckaufbau im Reaktor zu vermeiden. Gleichzeitig sollten die Katalysatorpartikel eine optimale Maximalgröße nicht überschreiten (D < 20 mm), um die Kontaktoberfläche zwischen dem Katalysator und dem Reaktionsgemisch zu erhöhen. Bevorzugt kommen die Schalenkatalysatoren zum Einsatz, in denen die Aktivkomponenten bevorzugt an der Oberfläche verteilt sind. Das Reaktionsgemisch wird mittels eines Wärmetauschers B abgekühlt und weiter dem Phasentrenner (Gas/Flüssigkeit Trenngefäß) C zugeführt. In diesem Behälter wird kontinuierlich, vorzugsweise bei niedriger Temperatur und erhöhtem Druck, die flüssige Phase von der Gasphase abgetrennt. Das Abgas kann entweder entsorgt oder vorzugsweise recycliert werden. Die flüssige Phase gelingt über Leitung 6 in den Behälter D, in dem pH durch Zugabe einer alkalischen Lösung (z.B. Natriumhydroxid in Methanol) über Leitung 7 auf einen pH-Wert zwischen 5 und 9 eingestellt wird. Ein Teil dieses Gemisches wird als Produkt über Leitung 9 abgetrennt, während der Rest über Leitung 8 zurück in den Reaktor A geführt wird.

In einer besonders bevorzugten Variante wird nicht das komplette Reaktionsgemisch mittels eines Wärmetauschers (B) nach dem Reaktor abgekühlt, sondern nur ein Teil davon, der nicht zurück in den Reaktor geführt wird. In diesem Fall (der Wärmetauscher B entfällt) wird das meiste Reaktionsgemisch bei einer Reaktionstemperatur recycliert und nur ein Teil davon über einen Wärmetauscher (E) als Produkt abgeführt und abgekühlt.

Anstelle von Festbettreaktor kann aber auch ein anderer z.B. Rührreaktor eingesetzt werden. Die Katalysatorpartikelgroße richtet sich dabei nach dem Reaktortyp. Bei einem Slurry-bed Reaktor wird z.B. ein Pulverkatalysator mit Partikelgroße < 0,2 mm eingesetzt.

### Beispiele

### Katalysatorherstellung

Katalysator 1 (0,9%Au-1,1%NiO auf SiO₂-Al₂O₃-MgO, 1,16-2,36 mm Kugel)
Eine Lösung von 37,5 g Aluminiumnitrat-Nonahydrat, 25,6 g Magnesiumnitrat-Hexahydrat und 5,4 g 60 % Salpetersäure in 100 mL Wasser wurde bei Raumtemperatur mit 108 g eines SiO2-Trägers (Fuji Silicia, Cariact Q-10, 1.16-2.36 mm Kugeln) vermischt. Das Gemisch wurde 24 h bei 50 °C gerührt, danach auf Raumtemperatur abgekühlt, bei 130 °C getrocknet und insgesamt 10 h bei 300 bis 600 °C kalziniert. 30 g dieses SiO₂-Al₂O₃-MgO Trägers wurden mit 100 mL Wasser vermischt und auf 90 °C aufgeheizt. Zu diesem Gemisch wurde nach 15 min bei 90 °C eine Lösung von 1,64 g Nickelnitrat-Hexahydrat und 530 mg Goldsäure (HAuCl₄) in 100 mL Wasser innerhalb von 30min zugegeben. Nach weiteren 30 min Rühren bei 90 °C wurde abgekühlt und der Feststoff abgetrennt, anschließend noch dreimal mit 100 mL frischem Wasser jeweils 5 min bei 20 °C gerührt und abfiltriert. Der Katalysator wurde bei 105 °C innerhalb von 10 h getrocknet und bei 450 °C innerhalb von 5 h an der Luft kalziniert. Der so erhaltene Katalysator enthielte nach ICP Analyse (Massenspektroskopie mit induktiv gekoppeltem Plasma) 1,1% Ni und 0,9% Au. Die mittlere Partikelgröße von Gold Nanopartikeln (TEM) betrug weniger als 5 nm.

### Beispiele

Eine Anlage mit kontinuierlicher Zugabe einer NaOH-Lösung und Rückführung eines Teils des Produktgemisches wurde bei dem folgenden Beispiel eingesetzt.

Das Reaktionsgemisch aus Methacrolein und Methanol (30,9 Gew% /69,1 Gew%) wurde mittels einer 1%igen NaOH-Lösung in Methanol auf pH = 7 eingestellt. Dieses neutralisierte Gemisch wurde mit einer Flowrate von 20,9 g/h zusammen mit einem O₂/N₂ Gasgemisch (7 vol% O₂) bei 11 bar über eine Leitung einem mit Außenmantel auf 70 °C beheizten Rohrreaktor zugeführt. Der O₂/N₂ Fluss wurde so eingestellt, dass der Anteil von O₂ im Abgas 4 Vol% betrug.Der Reaktor enthielte 15 g des Katalysators 1. In dem Neutralisationsgefäß D wurde pH = 7 mittels kontinuierlicher Addition von 1 %iger NaOH-Lösung in Methanol eingestellt. Das Verhältnis zwischen dem über Leitung 8 zurückgeführten Strom und dem Produktstrom betrug U/P = 0 bis 10 (siehe Tabelle). Das Produkt wurde zu bestimmten Laufzeiten der Anlage (siehe Tabelle) entnommen und mittels GC analysiert.

**Tabelle 1**

| Nr | Rücklauf [%] | Laufzeit [h] | U (MAL) [%] | RZA [mol MMA/kg cat h] | S(MMA) [%] | S(DMI) [%] |
|---|---|---|---|---|---|---|
| 1 | 90,9 | 73 | 70,8 | 4,12 | 97,4 | 0,1 |
| | 90,9 | 512 | 69,8 | 4,06 | 97,2 | 0,1 |
| 2 | 75 | 70 | 55,4 | 3,33 | 94,3 | 0,6 |
| | 75 | 250 | 53,2 | 3,20 | 94,1 | 0,6 |
| 3 | 0 | 24 | 34,5 | 2,15 | 90,9 | 5,2 |
| | 0 | 180 | 32,3 | 2,01 | 84,4 | 11,7 |

Wie aus der Tabelle 1 ersichtlich ist, erlaubt eine Ausführung wie oben abgebildet, den DMI-Anteil im Produkt konstant auf einem niedrigen Niveau zu halten, hohe MMA Selektivität und Aktivität des Katalysators bei einem hohem MAL Umsatz zu erzielen. Im Gegensatz dazu, wenn keine Rückführung angewendet ist (wie bei Nr. 3 in der Tabelle 1), wird viel DMI gebildet, die Selektivität zu MMA ist dementsprechend geringer und die RZA (Raum-Zeit-Ausbeute) des Katalysators sinkt.

### Zeichnungsindex

(A) Reaktor
(B) Wärmetauscher
(C) Phasentrenner
(D) Behälter zur Einstellung des pH-Werts
(E) Alternativer oder zusätzlicher Wärmetauscher
   (1) Zuleitung des O₂-haltigen Gasgemischs (10)
   (2) Zuleitung des Gemischs aus Methacrolein und Methanol (12)
   (3) Leitung zur Vermischung der flüssigen Phase (12) und der Gasphase (10) zur Einleitung in den Reaktor (A)
   (4) Leitung zur Überführung der entnommen flüssigen Phase aus Reaktor (A) in den Phasentrenner (C) über den Wärmetauscher (B)
   (5) Ableitung des Abgases (13) aus Phasentrenner (C)
   (6) Leitung für die flüssige Phase aus Phasentrenner (C) in Behälter (D)
   (7) Zuleitung der basischen Lösung (11) zur pH-Wert-Einstellung in Behälter (D)
   (8) Rückführung der pH-Wert eingestellten flüssigen Phase aus Behälter (D) zurück in Reaktor (A) über Leitung (3) und unter Anreicherung (1) mit Sauerstoff aus Gasgemisch (10)
   (9) Abführung des MMA-haltigen Produktstroms (14) zur weiteren Aufarbeitung

## Patentansprüche

1. Verfahren zur Herstellung von Methylmethacrylat aus Methacrolein in einer kontinuierlichen oxidativen Veresterungsreaktion mit Sauerstoff und Methanol, die in flüssiger Phase bei einem Druck von 2 bis 100 bar mit einem heterogenen Katalysator, bei dem es sich um geträgerte goldhaltige Nanopartikel mit einer Teilchengröße kleiner 20 nm handelt, durchgeführt wird, **dadurch gekennzeichnet,**
**dass** die flüssige Phase dem Reaktor kontinuierlich entnommen wird und der pH-Wert nach der Entnahme mittels Zugabe basischer Lösung außerhalb des Reaktors auf einen pH-Wert zwischen 5 und 9 eingestellt wird, und dass die entnommene flüssige Phase mit einem pH-Wert zwischen 5 und 9 zu mindestens 50 % zurück in den Reaktor geführt wird.

2. Verfahren gemäß Anspruch 1, **dadurch gekennzeichnet, dass** die entnommene flüssige Phase mit einem pH-Wert zwischen 5 und 9 zu mindestens 70 % zurück in den Reaktor geführt wird.

3. Verfahren gemäß Anspruch 1, **dadurch gekennzeichnet, dass** die entnommene flüssige Phase mit einem pH-Wert zwischen 5 und 9 zu mindestens 90 % zurück in den Reaktor geführt wird.

4. Verfahren gemäß Anspruch 1, **dadurch gekennzeichnet, dass** sich die goldhaltigen Nanopartikel auf Trägerpartikeln mit einem Gesamtdurchmesser zwischen 0,2 und 20 mm befinden, und dass das Verfahren in einem Festbettreaktor durchgeführt wird.

5. Verfahren gemäß Anspruch 1, **dadurch gekennzeichnet, dass** sich die goldhaltigen Nanopartikel auf Trägerpartikeln mit einem Gesamtdurchmesser kleiner 0,2 mm befinden, und dass das Verfahren in einem Reaktor mit Rührvorrichtung durchgeführt wird.

6. Verfahren gemäß mindestens einem der Ansprüche 1 bis 5, **dadurch gekennzeichnet, dass** die oxidative Veresterungsreaktion bei einem Druck im Bereich von 2 bis 50 bar und einer Temperatur im Bereich von 10 bis 200 °C durchgeführt wird.

7. Verfahren gemäß mindestens einem der Ansprüche 1 bis 6, **dadurch gekennzeichnet, dass** das frisch eingespeiste Gemisch aus Methanol und Methacrolein mit einem Anteil des Methacrolein bezogen auf das Gemisch im Bereich zwischen 20 und 60 Gew%, bevorzugt zwischen 25 und 40 Gew% in den Reaktor geleitet wird.

8. Verfahren gemäß mindestens einem der Ansprüche 1 bis 7, **dadurch gekennzeichnet, dass** der Restanteil der dem Reaktor kontinuierlich entnommenen flüssigen Phase, der nicht zurück in den Reaktor geführt wird, entwässert wird und anschließend das an Wasser reduzierte, Methacrolein und Methanol enthaltende Gemisch zurück in den Reaktor geleitet wird.

9. Verfahren gemäß mindestens einem der Ansprüche 1 bis 8, **dadurch gekennzeichnet, dass** die flüssige Phase zu 100 % zurück in den Reaktor geführt wird, und dass an einer anderen Stelle des Reaktors flüssige Phase zur weiteren Aufarbeitung kontinuierlich, semikontinuierlich oder batchweise entnommen wird.

10. Verfahren gemäß mindestens einem der Ansprüche 1 bis 9, **dadurch gekennzeichnet, dass** die Sauerstoffkonzentration im Abgas aus dem System weniger als 8 Vol% beträgt.

11. Verfahren gemäß mindestens einem der Ansprüche 1 bis 10, **dadurch gekennzeichnet, dass** der pH-Wert der aus dem Reaktor entnommenen flüssigen Phase auf einen Wert zwischen 6 und 8,5, bevorzugt zwischen 6,5 und 8,0 eingestellt wird.

12. Verfahren gemäß mindestens einem der Ansprüche 1 bis 11, **dadurch gekennzeichnet, dass** das Methacrolein vor der Zuleitung in den Reaktor in Gegenwart von Methanol destillativ entwässert wird.

13. Verfahren gemäß mindestens einem der Ansprüche 1 bis 12, **dadurch gekennzeichnet, dass** der Festbettreaktor mit einem Katalysatorvolumen/Reaktorvolumen Verhältnis größer 0,2 betrieben wird.

## Claims

1. Process for preparing methyl methacrylate from methacrolein in a continuous oxidative esterification reaction with oxygen and methanol which is conducted in the liquid phase at a pressure of 2 to 100 bar with a heterogeneous catalyst comprising supported gold-containing nanoparticles having a particle size less than 20 nm, **characterized in that**
the liquid phase is withdrawn continuously from the reactor and the pH, after the withdrawal, is adjusted to a pH between 5 and 9 by means of addition of basic solution outside the reactor, and
the withdrawn liquid phase having a pH between 5 and 9 is conducted back into the reactor to an extent of at least 50%.

2. Process according to Claim 1, **characterized in that** the withdrawn liquid phase having a pH between 5 and 9 is conducted back into the reactor to an extent of at least 70%.

3. Process according to Claim 1, **characterized in that** the withdrawn liquid phase having a pH between 5 and 9 is conducted back into the reactor to an extent of at least 90%.

4. Process according to Claim 1, **characterized in that** the gold-containing nanoparticles are present on support particles having a total diameter between 0.2 and 20 mm, and **in that** the process is conducted in a fixed bed reactor.

5. Process according to Claim 1, **characterized in that** the gold-containing nanoparticles are present on support particles having a total diameter of less than 0.2 mm, and **in that** the process is conducted in a reactor with stirring apparatus.

6. Process according to at least one of Claims 1 to 5, **characterized in that** the oxidative esterification reaction is conducted at a pressure in the range from 2 to 50 bar and at a temperature in the range from 10 to 200°C.

7. Process according to at least one of Claims 1 to 6, **characterized in that** the freshly fed mixture of methanol and methacrolein is passed into the reactor with a proportion of methacrolein, based on the mixture, in the range between 20 and 60% by weight, preferably between 25 and 40% by weight.

8. Process according to at least one of Claims 1 to 7, **characterized in that** the residual proportion of the liquid phase withdrawn continuously from the reactor which is not conducted back into the reactor is dewatered, and then the reduced-water mixture containing methacrolein and methanol is passed back into the reactor.

9. Process according to at least one of Claims 1 to 8, **characterized in that** 100% of the liquid phase is conducted back into the reactor, and **in that** liquid phase for further workup is withdrawn continuously, semicontinuously or batchwise elsewhere in the reactor.

10. Process according to at least one of Claims 1 to 9, **characterized in that** the oxygen concentration in the offgas from the system is less than 8% by volume.

11. Process according to at least one of Claims 1 to 10, **characterized in that** the pH of the liquid phase withdrawn from the reactor is set to a value between 6 and 8.5, preferably between 6.5 and 8.0.

12. Process according to at least one of Claims 1 to 11, **characterized in that** the methacrolein is dewatered by distillation in the presence of methanol before being fed into the reactor.

13. Process according to at least one of Claims 1 to 12, **characterized in that** the fixed bed reactor is operated with a catalyst volume/reactor volume ratio greater than 0.2.

## Revendications

1. Procédé pour la préparation de méthacrylate de méthyle à partir de méthacroléine dans une réaction d'estérification oxydante continue avec de l'oxygène et du méthanol, qui est réalisée en phase liquide à une pression de 2 à 100 bars avec un catalyseur hétérogène, pour lequel il s'agit de nanoparticules supportées contenant de l'or présentant une grosseur de particule inférieure à 20 nm, **caractérisé en ce que** la phase liquide est prélevée en continu du réacteur et le pH après le prélèvement est réglé par addition d'une solution basique en dehors du réacteur à une valeur de pH entre 5 et 9 et **en ce que** la phase liquide prélevée présentant une valeur de pH entre 5 et 9 est recyclée à raison d'au moins 50% dans le réacteur.

2. Procédé selon la revendication 1, **caractérisé en ce que** la phase liquide prélevée présentant une valeur de pH entre 5 et 9 est recyclée à raison d'au moins 70% dans le réacteur.

3. Procédé selon la revendication 1, **caractérisé en ce que** la phase liquide prélevée présentant une valeur de pH entre 5 et 9 est recyclée à raison d'au moins 90% dans le réacteur.

4. Procédé selon la revendication 1, **caractérisé en ce que** les nanoparticules contenant de l'or se trouvent sur des particules support présentant un diamètre total entre 0,2 et 20 mm et **en ce que** le procédé est réalisé dans un réacteur à lit fixe.

5. Procédé selon la revendication 1, **caractérisé en ce que** les nanoparticules contenant de l'or se trouvent sur des particules support présentant un diamètre total inférieur à 0,2 mm et **en ce que** le procédé est réalisé dans un réacteur équipé d'un dispositif d'agitation.

6. Procédé selon au moins l'une quelconque des revendications 1 à 5, **caractérisé en ce que** la réaction d'estérification oxydante est réalisée à une pression dans la plage de 2 à 50 bars et à une température dans la plage de 10 à 200°C.

7. Procédé selon au moins l'une quelconque des revendications 1 à 6, **caractérisé en ce que** le mélange fraîchement injecté de méthanol et de méthacroléine est guidé dans le réacteur à une proportion de méthacroléine, par rapport au mélange, dans la plage entre 20 et 60% en poids, de préférence entre 25 et 40% en poids.

8. Procédé selon au moins l'une quelconque des revendications 1 à 7, **caractérisé en ce que** la proportion résiduelle de la phase liquide prélevée en continu du réacteur, qui n'est pas recyclée dans le réacteur, est déshydratée et le mélange réduit en eau, contenant de la méthacroléine et du méthanol, est ensuite recyclé dans le réacteur.

9. Procédé selon au moins l'une quelconque des revendications 1 à 8, **caractérisé en ce que** la phase liquide est recyclée à raison de 100% dans le réacteur et **en ce que** de la phase liquide pour le traitement ultérieur est prélevée en continu, de manière semi-continue ou par lots en un autre endroit du réacteur.

10. Procédé selon au moins l'une quelconque des revendications 1 à 9, **caractérisé en ce que** la concentration en oxygène dans l'effluent gazeux du système est inférieure à 8% en volume.

11. Procédé selon au moins l'une quelconque des revendications 1 à 10, **caractérisé en ce que** la valeur du pH de la phase liquide prélevée du réacteur est réglée à une valeur entre 6 et 8,5, de préférence entre 6,5 et 8,0.

12. Procédé selon au moins l'une quelconque des revendications 1 à 11, **caractérisé en ce que** la méthacroléine est déshydratée par distillation avant l'introduction dans le réacteur en présence de méthanol.

13. Procédé selon au moins l'une quelconque des revendications 1 à 12, **caractérisé en ce que** le réacteur à lit fixe est exploité à un rapport volume de catalyseur/volume de réacteur supérieur à 0,2.
